# EUROPEAN PATENT APPLICATION

(11) **EP 2 617 497 A1**
(43) Date of publication of application: **24.07.2013**
(21) Application number: 13151643.7
(22) Date of filing: 17.01.2013
(51) Int. Cl.: B06B 1/06, G10K 11/00, G01S 7/52

(54) **Probe for ultrasonic diagnostic apparatus and method of manufacturing the same**

(30) Priority: 19.01.2012 KR 20120006227
(71) Applicant: Samsung Medison Co., Ltd., Gangwon-do 250-870 (KR)
(72) Inventor: Jun, Sung Jae, Gyeongsangbuk-do (KR); Lee, Sung Jae, Seoul (KR); Kim, Ji Seon, Daegu (KR); Seo, Min Seon, Gyeongbuk (KR)
(74) Representative: Schmid, Wolfgang

(57) **Abstract**

A probe for an ultrasonic diagnostic apparatus includes a case, a piezoelectric member provided inside the case, at least one acoustic matching layer disposed at a front of the piezoelectric member, an acoustic lens disposed at a front of the acoustic matching layer, and a backing member disposed at a rear of the piezoelectric member and provided with a plurality of pores to reduce sound waves transmitted to the rear of the piezoelectric member and to allow to adjust an acoustic impedance. A method of manufacturing the probe for an ultrasonic diagnostic apparatus includes the operations of preparing a fluid to form a backing layer, pouring the fluid into a mold, hardening the fluid and removing the mold.

## Description

### BACKGROUND

### 1. Field

Exemplary embodiments of the present disclosure relate to a probe for an ultrasonic diagnostic apparatus which may increase sensitivity of the probe, and a method of manufacturing the same.

### 2. Description of the Related Art

An ultrasonic diagnostic apparatus is a noninvasive apparatus that transmits an ultrasound signal to a body surface of a subject towards a target organ in the body and obtains cross-sectional images of soft tissue and blood flow using information from a reflected ultrasound signal. Compared to other medical imaging systems such as, for example, X-ray imaging systems, computed tomography (CT) scanners, magnetic resonance imaging (MRI) systems and diagnostic systems for nuclear medicine, the ultrasonic diagnostic apparatus may have a compact size and low price, display images in real time, and provide a high level of safety by eliminating exposure to radiation such as X-rays. For at least these reasons, ultrasonic diagnostic apparatuses have been widely used for diagnosis in many medical disciplines such as, for example, cardiac medicine, abdominal imaging, urology, obstetrics and gynecology.

An ultrasonic diagnostic apparatus includes a probe which transmits an ultrasound signal to a subject and receives an ultrasound echo signal reflected from the subject to obtain an ultrasound image of the subject.

A probe for an ultrasonic diagnostic apparatus may include a transducer, a case with an open upper end, and a cover coupled to the open upper end of the case to directly contact a surface of the subject.

The transducer may include a piezoelectric layer composed of piezoelectric materials which converts an electric signal into an acoustic signal, and vice versa, through vibration of the piezoelectric materials, an acoustic matching layer for reducing a difference in acoustic impedance between the piezoelectric layer and the subject to improve the transferability of ultrasonic waves generated in the piezoelectric layer to the subject, a lens layer for focusing the ultrasonic waves traveling away from a front of the piezoelectric layer on a given point, and a backing member for blocking the ultrasonic waves from traveling in an opposite direction through a rear of the piezoelectric layer to prevent image distortion.

In order to increase attenuation of the acoustic impedance of the backing member, powder, a network sheet, and a porous sheet in combination with an epoxy resin has been used. However, in such a configuration, the powder may not be regularly distributed, and it may be difficult to produce powder with a uniform size. Further, pores that are unexpectedly produced inside the epoxy resin are a characteristic that is uncontrollable and not predetermined during mass production, and thus may cause efficiency to vary from one product to another.

### SUMMARY

Therefore, it is an object of the present disclosure to provide a probe for an ultrasonic diagnostic apparatus which may increase attenuation of the acoustic impedance of a backing member of the probe to enhance performance efficiency of the probe, and a method of manufacturing the same.

Additional aspects of the disclosure will be set forth in part in the description which follows and, in part, will be obvious from the description, or may be learned from practice of the disclosure.

In accordance with one aspect of the present disclosure, a probe for an ultrasonic diagnostic apparatus includes a case, a piezoelectric member provided inside the case, at least one acoustic matching layer disposed at a front of the piezoelectric member, an acoustic lens disposed at a front of the at least one acoustic matching layer, and a backing member disposed at a rear of the piezoelectric member and provided with a plurality of pores to reduce sound waves transmitted to the rear of the piezoelectric member and to adjust acoustic impedance.

The pores may be formed through the backing member.

The pores may be arranged in a predetermined pattern.

The backing member may further include at least one empty particle formed of glass with a hollow inside.

The backing member may include at least one empty particle formed of glass with a hollow inside to form the pores.

The backing member may include glass particles in a proportion over a certain proportion to control attenuation, hardness and density of the backing member.

Upper and lower sides of the piezoelectric member may be provided with an electrode to provide an electric signal to the piezoelectric member, and a printed circuit board (PCB) may be provided between the electrodes and the backing member to connect electrically the electrode and a main body of the ultrasonic diagnostic apparatus.

The backing member may include a backing layer positioned at a lower side of the PCB and a backing block positioned at a lower side of the backing layer.

In accordance with another aspect of the present disclosure, a method of manufacturing a backing member used for a probe for an ultrasonic diagnostic apparatus includes pouring a fluid forming the backing member into a mold including a mold base provided with sticks having a shape corresponding to pores to be provided in the backing member and a mold guide to form an outer shape of the backing member, hardening the fluid poured into the mold, and removing the mold.

The sticks provided in the mold base may be regularly arranged.

The fluid forming the backing member may include at least one hollow particle to form pores in the backing member.

The fluid forming the backing member may further include at least one glass particle.

The hardening may be performed at a temperature between about 30°C and about 100°C.

### BRIEF DESCRIPTION OF THE DRAWINGS

These and/or other aspects of the disclosure will become apparent and more readily appreciated from the following description of the embodiments, taken in conjunction with the accompanying drawings of which:
FIG. 1 is a view illustrating exterior of the probe for an ultrasonic diagnostic apparatus according to an exemplary embodiment of the present disclosure;
FIG. 2 is a perspective view illustrating a probe for an ultrasonic diagnostic apparatus according to an exemplary embodiment of the present disclosure;
FIG. 3 is an exploded view illustrating the probe for an ultrasonic diagnostic apparatus of FIG. 1;
FIG. 4 is a view illustrating a mold and a backing member used to manufacture the probe for an ultrasonic diagnostic apparatus of FIG. 1;
FIG. 5 is a view illustrating a backing member according to another embodiment of the present disclosure; and
FIG. 6 is a flowchart illustrating a process of manufacturing a backing member of a probe for an ultrasonic diagnostic apparatus according to one embodiment of the present disclosure.

### DETAILED DESCRIPTION

Reference will now be made in detail to exemplary embodiments of the present disclosure, examples of which are illustrated in the accompanying drawings, wherein like reference numerals refer to like elements throughout.

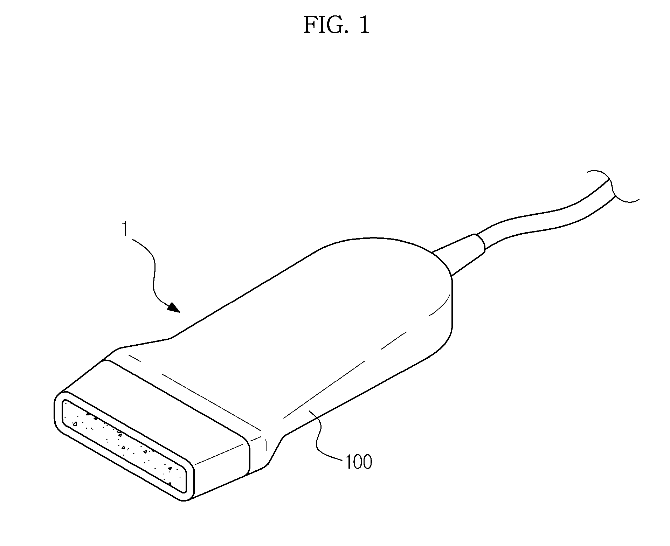

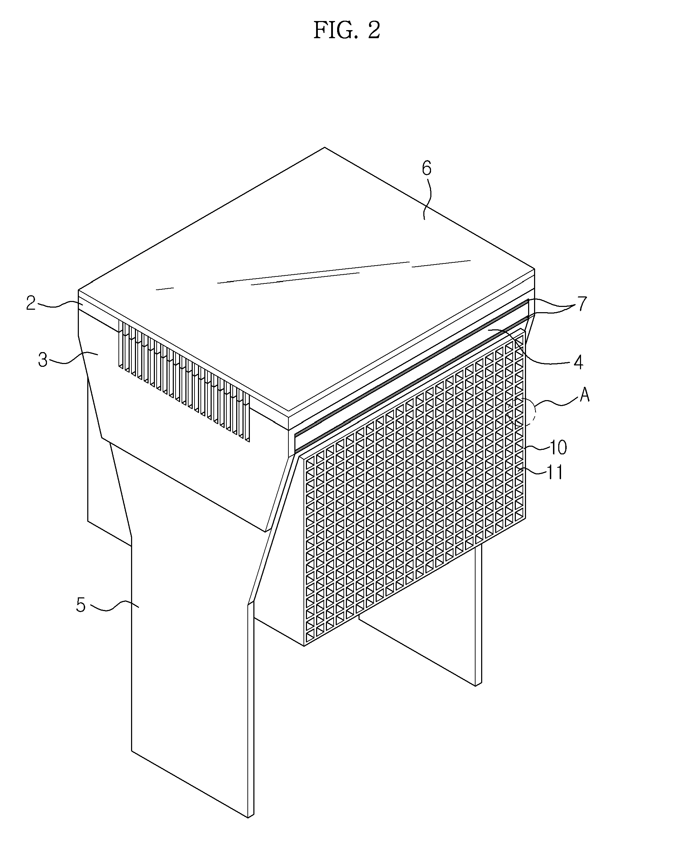

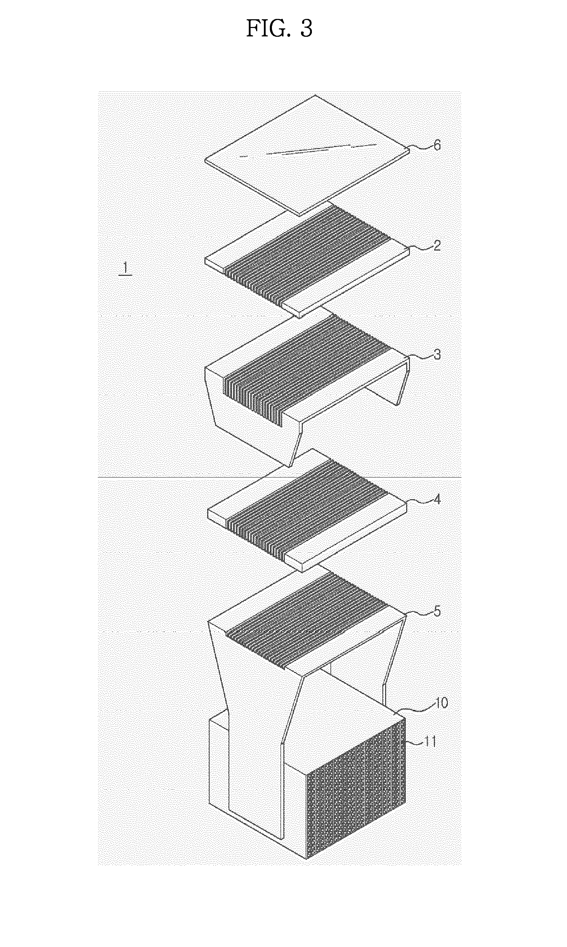

FIG. 1 is a view illustrating exterior of the probe for an ultrasonic diagnostic apparatus according to an exemplary embodiment of the present disclosure, FIG. 2 is a perspective view illustrating a probe for an ultrasonic diagnostic apparatus according to an exemplary embodiment of the present disclosure, and FIG. 3 is an exploded view illustrating the probe of FIG. 1.

As shown in FIG.'S 1, 2 and 3, a transducer includes a piezoelectric member 4, acoustic matching layers 2 and 3, an acoustic lens (6) and a backing member 10. The illustrated transducer may be a constituent of a probe 1 for an ultrasonic diagnostic apparatus according to an exemplary embodiment of the present disclosure, and in practice may be positioned inside a case (100).

The acoustic lens (6), acoustic matching layers 2 and 3, piezoelectric member 4 and backing member 10 may be arranged in this order from a front face of the transducer.

The piezoelectric member 4 may be positioned inside the case (100), and connected to a front surface of the backing member 10. The piezoelectric member 4 is provided with electrodes (7) on opposite lateral sides thereof for converting an electric signal into an ultrasonic wave, i.e., an acoustic signal which is sent into the air, and for converting an ultrasonic wave reflected from the air into an electric signal which is sent to the apparatus.

The piezoelectric member 4, which generates an ultrasonic wave using the phenomenon of resonance, may be composed of, for example, lead zirconate titanate (PZT) ceramics, PZNT single crystals made of a solid solution of lead zinc niobate and lead titanate, PZMT single crystals made of a solid solution of lead magnesium niobate and lead titanate, or the like, or a combination thereof.

The electrodes (7) disposed on opposite lateral sides of the piezoelectric member 4 may be composed of a metal with high conductivity such as, for example, gold, silver and/or copper, or may be composed of a graphite, or the like.

The acoustic matching layers 2 and 3 are installed on a front of the piezoelectric member 4. The acoustic matching layers 2 and 3 serve to match the acoustic impedance of the piezoelectric member 4 with that of a subject to effectively transfer an ultrasonic signal generated by the piezoelectric member 4 to the subject. To this end, the acoustic impedance of the acoustic matching layers 2 and 3 may be adapted to have a middle value between the acoustic impedances of the piezoelectric member 4 and the subject.

The acoustic matching layers 2 and 3 may be f composed of glass or a resin material, or a combination thereof. Alternately, a plurality of acoustic matching layers composed of different materials may be provided to allow the acoustic impedance to gradually vary from the piezoelectric member 4 to the subject. FIG.'S 2 and 3 show an exemplary embodiment in which a first acoustic matching layer 2 and a second acoustic matching layer 3 are provided, but embodiments of the present disclosure are not limited thereto.

A printed circuit board (PCB) 5 may be arranged between the backing member 10 and the piezoelectric member 4. The PCB 5 is provided to convert an electric signal generated at the electrodes (7) into an ultrasonic signal and vice versa. The PCB 5 may be disposed perpendicular to the direction in which the backing member 10 and the piezoelectric member 4 are stacked. In addition to a printed circuit board, the PCB 5 may include other constituents such as, for example, a flexible printed circuit board (FPCB) adapted to supply signals or electricity.

An acoustic lens (6) is disposed on a front of the acoustic matching layers 2 and 3. The acoustic lens (6) focuses an ultrasonic signal traveling forward from the piezoelectric member 4 on a given point.

The backing member 10 is disposed at a rear of the piezoelectric member 4. The backing member 10 reduces a pulse width of an ultrasonic wave by suppressing free vibration of the piezoelectric member 4, and prevents image distortion by blocking unnecessary propagation of the ultrasonic wave in a rearward direction of the piezoelectric member 4.

The backing member 10 includes pores 11 to reduce the density of the backing member 10. The pores 11 not only reduce the density of the backing member 10, but also reduce sound waves transferred to the backing member 10. The pores 11 may be formed through the backing member 10. Also, the pores 11 may be arranged in a predetermined pattern.

According to one exemplary embodiment of the present disclosure, the pores 11 are arranged in lengthwise and widthwise directions with a uniform interval there between. However, embodiments of the present disclosure are not limited thereto. The pores 11 may be arranged with a narrower and/or wider interval there between than the interval shown in FIG.'S 2 and 3.

FIG.'S 2 and 3 illustrate that the pores 11 have a rectangular shape, but embodiments of the present disclosure are not limited thereto. It may be possible for the pores 11 to have other shapes such as, for example, a circular shape .

The shape and interval of the pores 11 may vary depending on the shape of a mold base 30, which will be described later.

Since the backing member 10 includes the pores 11, the density of the backing member 10 is lowered, which is related to acoustic impedance. The acoustic impedance is an indicator of the acoustic characteristics of a medium through which sound travels. When the acoustic impedance is Z, the density of the medium is p, and sound speed is C, the acoustic impedance is expressed by the following equation.

Acoustic impedance (Z) = Density of medium (p) + Sound speed (C).

For the backing member 10 to effectively prevent an ultrasonic wave from being unnecessarily propagated in the rearward direction of the piezoelectric member 4, the acoustic impedance of the backing member 10 should be adjusted for a particular application. Accordingly, the backing member 10 should be manufactured such that the acoustic impedance of the backing member 10 is high or low according to the acoustic design. When the backing member 10 includes the pores 11 as in the present disclosure, the density of the backing member 10 may be adjusted by adjusting the number of pores 11 in the backing member 10. Thereby, as the acoustic impedance of the backing member is allowed to be adjusted as intended by the designer, a flexible design may be enabled in terms of acoustic impedance. Further, the pores 11 of the backing member 10 may serve to absorb vibration generated by the piezoelectric member 4.

The backing member 10 may be composed of a material including an epoxy resin.

As shown in FIG.'S 2 and 3, the backing member 10 may be formed into a backing block, and may include a backing layer in addition to the backing block. The backing layer is provided to support the piezoelectric member 4 when, for example, the probe 1 is of a convex type which has a convex surface. The backing layer may also be provided with pores 11 as provided in the backing block to increase attenuation of the acoustic impedance of the backing layer.

The probe 1 may be of a linear type which has a linear surface, or of the convex type which has a convex surface formed in a curved shape.

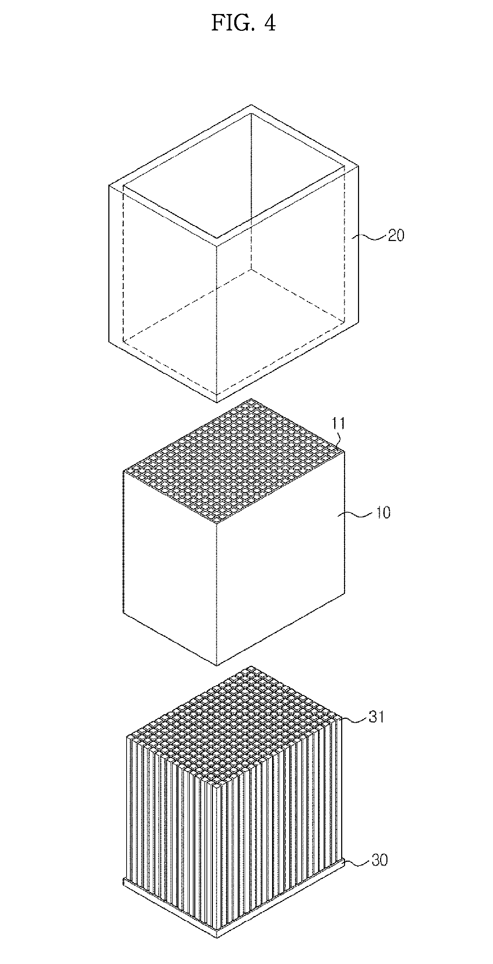

FIG. 4 shows a mold and a backing member used to manufacture the probe for an ultrasonic diagnostic apparatus according to the illustrated embodiment.

As shown in FIG. 4, the backing member 10 may be manufactured using a mold including a mold base 30 and a mold guide 20. The mold base 30 is provided with a plurality of sticks 31 to form the pores 11 in the backing member 10. The mold guide 20, which is a frame to surround the mold base 30, determines the outer appearance of the backing member 10.

The upper and lower sides of the mold guide 20 have openings, and the mold base 30 is coupled with the mold guide 20 to form a bottom. A fluid to form the backing member 10 is poured into the opening on the upper side of the mold guide 20.

The sticks 31 of the mold base 30 may be provided in various shapes and with various arrangements, and the pores 11 of the backing member 10 may be formed in various shapes and with various arrangements that are predetermined. Because the pores 11 are defined by the sticks 31 of the mold base 30, the pores 11 of the backing member 10 may be regularly arranged. Accordingly, when the pores 11 of the backing member 10 are made using the mold (e.g., mold guide 20 and mold base 30), the pores 11 may be provided with a regular arrangement, pattern and shape that are predetermined in contrast with conventional cases in which powder was used. Since the arrangement, pattern and shape of the backing member 10 may be altered by changing the shape, arrangement and pattern of the sticks 31 of the mold base 30, it may be possible to provide a proper impedance value to the backing member 10 according to purpose.

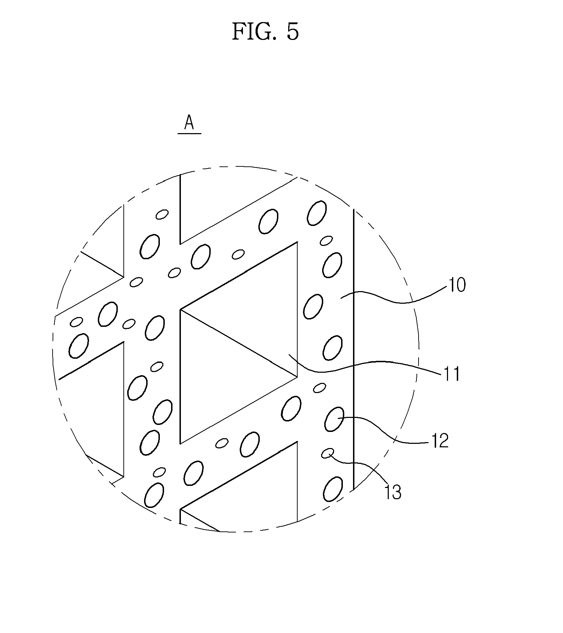

FIG. 5 is an enlarged view illustrating a backing member according to another embodiment of the present disclosure.

FIG. 5 shows an enlarged perspective view of section A of FIG. 3. The backing member 10 shown in FIG. 5 also includes pores 11 as in the embodiment shown in FIG.'S 2 to 4, and it further includes additional pores 12.

The pores 12 may include particles formed of glass with a hollow inside. Since the insides of the particles 12 are empty, the particles 12 may increase attenuation of a sound wave propagated through the backing member 10.

Further, if the empty particles 12 and glass particles 13 are mixed in a certain ratio, it may be possible to control the degree of attenuation, hardness, and density of the backing member 10 simultaneously. That is, it may be possible to adjust the acoustic impedance.

When the pores 12 are formed using the empty particles, the backing member 10 may have a low density and provide more effective attenuation of ultrasonic waves.

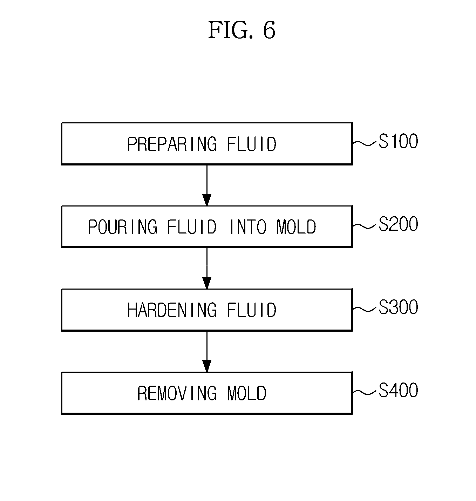

FIG. 6 is a flowchart illustrating an exemplary process of manufacturing a backing member of a probe for an ultrasonic diagnostic apparatus according to one embodiment of the present disclosure.

As shown in FIG. 6, the backing member 10 is manufactured through the operations of preparing a fluid (S100), pouring the fluid into a mold (S200), hardening the fluid (S300) and removing the mold (S400).

In addition to the operations above, one or more additional operations may be added by one of ordinary skill in the art.

The operation of preparing a fluid (S100) includes preparing an epoxy resin which is a material used for the backing member 10. Also, when including the additional pores 12 shown in FIG. 5, empty particles 12 may be included. Further, in addition to the empty particles 12, the glass particles 13 may be added to manufacture a backing member having an estimated degree of attenuation, hardness and density (i.e., acoustic impedance).

The operation of pouring the prepared fluid into a mold (S200) includes pouring the fluid into the mold configured with the mold guide 20 and the mold base 30. The mold guide 20 and the mold base 30 are allowed to be coupled with each other, and the mold base 30 is provided with the sticks 31 having a shape corresponding to that of the pores 11 to be provided in the backing member 10. The sticks 31 provided at the mold base 30 may be regularly arranged and the pores 11 formed in the backing member 10 are provided with a shape corresponding to the sticks 31 of the mold base 30.

In the operation of hardening the mold (S300), the fluid in the mold is hardened in the shape of the backing member 10 with the pores 11. The fluid is hardened at a temperature between about 30°C and about 100°C.

Thereafter, the operation of removing the mold (S400) is performed, and the backing member 10 is manufactured. The backing member 10 with the pores 11 regularly arranged may be obtained by removing the mold guide 20 and the mold base 30.

As is apparent from the above description, because of pores provided in a backing member, efficiency of a probe for an ultrasonic diagnostic apparatus may be increased by increasing attenuation of the acoustic impedance of a backing member, and a backing member with low density and low weight may be manufactured.

Although a few embodiments of the present disclosure have been shown and described, it would be appreciated by those skilled in the art that changes may be made in these embodiments without departing from the principles and spirit of the disclosure, the scope of which is defined in the claims and their equivalents.

## Claims

1. A probe for an ultrasonic diagnostic apparatus comprising:
a case;
a piezoelectric member provided inside the case;
at least one acoustic matching layer disposed at a front of the piezoelectric member;
an acoustic lens disposed at a front of the at least one acoustic matching layer; and
a backing member disposed at a rear of the piezoelectric member and provided with a plurality of pores to reduce sound waves transmitted to the rear of the piezoelectric member and to adjust acoustic impedance.

2. The probe according to claim 1, wherein the pores are formed through the backing member.

3. The probe according to claim 2, wherein the pores are arranged in a predetermined pattern.

4. The probe according to claim 3, wherein the backing member further includes at least one empty particle formed of glass with a hollow inside.

5. The probe according to claim 1, wherein the backing member includes at least one empty particle formed of glass with a hollow inside to form the pores.

6. The probe according to claim 5, wherein the backing member further includes glass particles in a proportion over a certain proportion to control attenuation, hardness and density of the backing member.

7. The probe according to claim 1, wherein upper and lower sides of the piezoelectric member are provided with an electrode to provide an electric signal to the piezoelectric member, and a printed circuit board (PCB) is provided between the electrodes and the backing member to connect electrically the electrode and a main body of the ultrasonic diagnostic apparatus.

8. The probe according to claim 7, wherein the backing member includes a backing layer positioned at a lower side of the PCB and a backing block positioned at a lower side of the backing layer.

9. A method of manufacturing a backing member used for a probe for an ultrasonic diagnostic apparatus, comprising:
pouring a fluid forming the backing member into a mold including a mold base provided with sticks having a shape corresponding to pores to be provided in the backing member and a mold guide to form an outer shape of the backing member;
hardening the fluid poured into the mold; and
removing the mold.

10. The method according to claim 9, wherein the sticks provided in the mold base are regularly arranged.

11. The probe according to claim 9, wherein the fluid forming the backing member includes at least one empty particle to form pores in the backing member.

12. The method according to claim 11, wherein the fluid forming the backing member further includes at least one glass particle.

13. The method according to claim 11, wherein the hardening is performed at a temperature between about 30ºC and about 100ºC.
